# EUROPEAN PATENT SPECIFICATION

(11) **EP 1 778 359 B1**
(45) Date of publication and mention of the grant of the patent: **13.10.2010**
(21) Application number: 05769771.6
(22) Date of filing: 31.07.2005
(51) Int. Cl.: A61K 36/736, A61K 36/752, A61P 3/10

(54) **PHARMACEUTICAL COMPOSITIONS FOR ALLEVIATING EXCESS LEVELS OF SUGAR IN DIABETIC PATIENTS**
PHARMAZEUTISCHE ZUSAMMENSETZUNGEN ZUR LINDERUNG VON ÜBERMÄSSIG HOHEN ZUCKERSPIEGELN BEI DIABETIKERN
COMPOSITIONS PHARMACEUTIQUES PERMETTANT D'ATTENUER LES NIVEAUX EXCESSIFS DE SUCRE CHEZ LES PATIENTS DIABETIQUES

(30) Priority: 08.08.2004 IL 16340904; 07.07.2005 IL 16957805
(43) Date of publication of application: 02.05.2007
(73) Proprietor: KHAYAT, Eli, 25123 Neve Ziv (IL); SINAI, Yuval Mashiach, 93840 Gilo, Jerusalem (IL)
(72) Inventor: KHAYAT, Eli, 25123 Neve Ziv (IL); SINAI, Yuval Mashiach, 93840 Gilo, Jerusalem (IL)
(74) Representative: Schwahn, Hartmut
(86) International application number: PCT/IL2005/000811
(87) International publication number: WO 2006/016352

(56) References cited:
- US-A1- 2003 072 824
- DATABASE WPI Section Ch, Week 200264 Derwent Publications Ltd., London, GB; Class B04, AN 1998-340637 XP002355757 & JP 03 326589 B2 (KAMEYAMA Y) 24 September 2002 (2002-09-24)
- DATABASE WPI Section Ch, Week 200207 Derwent Publications Ltd., London, GB; Class B04, AN 2002-049847 XP002355758 & CN 1 306 834 A (HIGH QUALITY FARM PROD DEV SERVICE CENT) 8 August 2001 (2001-08-08)
- GRINDLEY PHILLIP B A ET AL: "Carbohydrate digestion and intestinal ATPases in streptozotocin-induced diabetic rats fed extract of yam (Dioscorea cayenensis) or dasheen (Colocasia esculenta)" NUTRITION RESEARCH, vol. 22, no. 3, March 2002 (2002-03), pages 333-341, XP002355754 ISSN: 0271-5317
- STACEWICZ-SAPUNTZAKIS M ET AL: "CHEMICAL COMPOSITION AND POTENTIAL HEALTH EFFECTS OF PRUNES: A FUNCTIONAL FOOD?" CRITICAL REVIEWS IN FOOD SCIENCE AND NUTRITION, BOCA RATON, FL, US, vol. 41, no. 4, May 2001 (2001-05), pages 251-286, XP009056029 ISSN: 1040-8398
- MICKLANDER E ET AL: "NONINVASIVE ASSAY FOR CYANOGENIC CONSTITUENTS IN PLANTS BY RAMAN SPECTROSCOPY: CONTENT AND DISTRIBUTION OF AMYGDALIN IN BITTER ALMOND (PRUNUS AMYGDALUS)" APPLIED SPECTROSCOPY, THE SOCIETY FOR APPLIED SPECTROSCOPY. BALTIMORE, US, vol. 56, no. 9, September 2002 (2002-09), pages 1139-1146, XP001125067 ISSN: 0003-7028

## Description

The present invention provides a composition for alleviating excessive levels of sugar in the blood of type 2 diabetic patients. More particularly the present invention relates to a pharmaceutical composition for alleviating excessive levels of sugar in the blood of type 2 diabetic patients utilizing one or more active ingredients, wherein at least one of said active ingredients is a cyanogenic glucoside from a natural source.

The number of people with diabetes is growing to epidemic proportions in the developed countries. There is a great deal of research on the evolving understanding of the pathogenesis of diabetes as compared to normoglycemia. The diagnostic criteria for diabetes have become streamlined to more appropriately and accurately diagnose the disease. There are millions of people who have diabetes, but do not know it. It is essential that appropriate screening be performed to make a diagnosis in order to delay or prevent the complications from occurring. The complications of diabetes have implications for the increasing number of people with the diagnosis who are hospitalized and how they are treated. There are specific methods for recognition and treatment of both acute and chronic complications in the hospitalized patent with diabetes. Managing blood glucose control is essential for favorable outcomes.

Type II diabetes typically occurs in individuals older than 40 years who have a family history of diabetes. Type 2 diabetes is characterized by peripheral insulin resistance with an insulin-secretory defect that varies in severity. These defects lead to increased hepatic gluconeogenesis, which produces fasting hyperglycemia. Most patients (90%) who develop type 2 diabetes are obese, and obesity itself is associated with insulin resistance, which worsens the diabetic state. Since patients with type 2 diabetes retain the ability to secrete some endogenous insulin, those who are taking insulin do not develop DKA if for some reason they stop taking it. Therefore, they are considered to require insulin but not to depend on insulin. Moreover, patients with type 2 diabetes often do not need treatment with oral antidiabetic medication or insulin if they lose weight by successfully adhering to a physician-directed weight loss program including strict diet control and exercise.

A variety of other types of diabetes, previously called "secondary diabetes," are caused by other illnesses or medications. Depending on the primary process involved (i.e, destruction of pancreatic beta cells or development of peripheral insulin resistance), these types of diabetes behave similarly to type 1 or type 2 diabetes. The most common are diseases of the pancreas that destroy the pancreatic beta cells (e.g., hemochromatosis, pancreatitis, cystic fibrosis, pancreatic cancer); hormonal syndromes that interfere with insulin secretion (e.g., pheochromocytoma) or cause peripheral insulin resistance (e.g., acromegaly, Cushing syndrome, pheochromocytoma); and drug-induced diabetes (e.g., phenytoin, glucocorticoids, estrogens).

Maturity-onset diabetes of the young (MODY) is a form of type 2 diabetes that affects many generations in the same family with onset in individuals younger than 25 years. Several types exist.

Gestational diabetes mellitus (GDM) is defined as any degree of glucose intolerance with onset or first recognition during pregnancy. GDM complicates approximately 4% of all pregnancies in the US, although this ranges from 1-14% depending on the population studied. Untreated GDM can lead to fetal macrosomia, hypoglycemia, hypocalcemia, and hyperbilirubinemia. In addition, mothers with GDM have higher rates of cesarean delivery and chronic hypertension. To diagnose GDM, a 50-gram glucose screening test should be done at 24-28 weeks of gestation. This is followed by a 100-gram, 3-hour oral glucose tolerance test if the 1-hour postscreen plasma glucose concentration is greater than 140 mg/dL.

In the US, 10.3 million people have been diagnosed with diabetes and an estimated additional 5.4 million have undiagnosed diabetes. Approximately 90% have type 2 diabetes and the remainder have type 1 diabetes.

The morbidity and mortality associated with diabetes are related to the short-and long-term complications. These complications include hypoglycemia and hyperglycemia, increased risk of infections, microvascular complications (i.e., retinopathy, nephropathy), neuropathic complications, and macrovascular disease. Diabetes is the major cause of blindness in adults aged 20-74 years, as well as the leading cause of nontraumatic lower-extremity amputation and end-stage renal disease (ESRD).

Type 2 diabetes mellitus is more prevalent among Hispanics (10.6%), Native Americans (12-50%, depending on the tribe), African Americans (10.8%), and Asians/Pacific Islanders than in whites.

Incidence is essentially equal in females and males in all populations.

Type 2 diabetes is becoming increasingly common because more people are living longer (diabetes prevalence increases with age). It is also being seen more frequently in younger people in association with the rising prevalence of childhood obesity. Although type 2 diabetes still occurs most commonly in adults aged 40 years and older, the incidence of disease is increasing more rapidly in adolescents and young adults than in other age groups.

With this state of the art in mind there is now provided according to the present invention a pharmaceutical composition for alleviating excess levels of sugar in the blood of type 2 diabetic patients comprising a source of at least one cyanogenic glucoside as active ingredient therein.

In the present invention said source is a natural source.

In the present invention said natural source of at least one cyanogenic glucoside is from kernels of bitter almonds.

In further preferred embodiments of the present invention said natural source of at least one cyanogenic glucoside is from kernels from fruits of the *Prunus* species.

In a most preferred embodiment of the present invention there is provided a pharmaceutical composition for alleviating excess levels of sugar in the blood of type 2 diabetic patients comprising a powder formed from a mixture of dry bitter almond kernels and from a dried lemon component selected from the group consisting of lemon pulp, peel, and seeds and combinations thereof.

In these preferred embodiments said powder preferably comprises between about 70% and 90% dried and ground bitter almond kernels, and between about 30% and 10% of a dried lemon component selected from the group consisting of lemon pulp, peel, and seeds and combinations thereof.

In yet further embodiments the present invention is directed to the use of a source of at least one cyanogenic glucoside according to the claims for the manufacture of a pharmaceutical composition for alleviating excess levels of sugar in the blood of type 2 diabetic patients substantially as described herein.

As stated in its most preferred embodiment the present invention is directed to pharmaceutical compositions containing as active ingredients therein compounds found in bitter almonds kernels and or leaves and in lemon pulp, peel or seeds. Both components are dried up by a hot air stream and mixed in a ratio of 1:4 (lemon/almonds respectively). The daily dose for an adult type 2 diabetic patient is preferably about 15,000 mg. although other doses can also be utilized. The dry powder is administered orally 3 times a day. Each of the components is capable of lowering blood sugar in diabetic patients but the combined formulation is more effective than each individually. The powder may be orally administered as a powder or as a suspension in water or other non-sugared liquids. The daily dose ranges according to age, body weight and degree of illness. Nevertheless a 15,000 mg per day per adult person is the recommended dose. The formulation may be administered as a non-insulin dependent additive.

It is most effective in patients that can synthesis approximately 70% or higher of the medically considered normal levels of insulin.

It is highly effective in overweight patients that synthesize normal or close to normal amounts of insulin.

The fact that both the kernels of bitter almonds and the lemon tissues contain cyanogenic glucosides along with other circumstantial results suggests that CN-glucoside is an effective compound for lowering blood sugar levels in diabetic patients. Kernels of other *Prunus* species contain cyanogenic glucosides and consequently were found effective as well to lower blood sugar and are therefore included within the scope of the present invention.

It is to be noted that amygdalin otherwise known as d-Mandelonitrile glucoside or as amygdaloside is recognized as a major glucoside from bitter almonds and was used in some of the examples herein.

While in preferred embodiments of the present invention and in the following examples, a natural source of at least one cyanogenic glucoside was used and tested, the invention is also directed to the utilization of synthetic sources thereof.

Furthermore, in other preferred embodiments of the present invention, other components as set forth in tables 1 and 2 hereinafter, can be included in the pharmaceutical composition to act in a complimentary or other function together with the cyanogenic glucoside.

While the invention will now be described in connection with certain preferred embodiments in the following examples and with reference to the accompanying figures so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

In the drawings:
Figure 1 is a graphical representation of the mean distribution of glucose levels in blood as a function of time after the administration of a composition according to the present invention and administration of a placebo (sweet almonds).
Figure 2A is a graphical representation of an HPLC analysis of amygdalin in.
Figure 2B is a graphical representation of an HPLC analysis of amygdalin in sweet almonds; and.
Figure 2C is a graphical representation of an HPLC analysis of a standard commercial amygdalin.

### Example 1

A 58 year old female patient who suffers from diabetes type II (N.I.D.D.M.) since the age of three and who was treated previously with glucophage tablets 1 x 3 per day for a few months stopped taking said tablets and instead took 1 teaspoon three times a day of a powder according to the present invention containing 85% ground dry bitter almond kernels and 15% ground dried lemon pulp, peel and seeds.

The last blood test of this patient was carried out six months before treatment and all were in the normal range, with the exception of the blood sugar of this patient which was ranging between 280 mg% and 300 mg% prior to treatment. After treatment with the powder of the present invention for two months the average level of blood sugar dropped to 120 mg% on fasting and 170 mg% after a meal.

### Example 2

A 64 year old male patient who suffers from diabetes type II (N.I.D.D.M.) since the age of five and who was treated previously with glucophage tablets once a day and glucomine once a day, was found to have no other diseases and no signs of diabetic complications. His last blood tests were done in April 2004 and were all in the normal range, with the exception of average blood sugar which was found to be in the range of 170-200 mg%.

He began taking 1 teaspoon three times a day of a powder according to the present invention containing 85% ground dry bitter almond kernels and 15% ground dried lemon pulp, peel and seeds, in combination with one tablet a day of the glucophage tablet and one tablet a day of the glucomine for one month and then continued taking 1 teaspoon three times a day of the powder according to the present invention and only half a tablet a day of the glucophage tablet and half a tablet a day of the glucomine and continued this regimen for an additional three months. At the end of this four month period the average blood sugar of this patient was down to 140-150 mg%

### Example 3

A 62 year old male patient who suffers from diabetes type II (N.I.D.D.M.) for the last 14 years and who was treated previously with one glucophage tablet three times a day stopped taking said tablets and began taking one teaspoon three times a day of a powder according to the present invention containing 85% ground dry bitter almond kernels and 15% ground dried lemon pulp, peel and seeds,

This patient suffers from hypertension - treated with normiten, angina pectoris - not active and not treated, hypercholesterolemia and hypertrigliceridemia treated with simovil and asthma which is not active.

Before treatment the average blood sugar was 285 mg% and following the treatment the level of blood sugar dropped to 110 -140 mg%.

As will be noted with each of the three patients there was a reduction of blood sugar levels attributable to treatment with a composition according to the present invention, despite the reduction or complete cessation of the conventional medical treatment.

### Example 4 The metabolic effect of bitter almonds-based mixture in patients with Type 2 diabetes

A clinical experiment was carried out at the Diabetes Unit of the Hadassah Medical Organization at Hadassah Hospital Ein Kerem Jerusalem, in order to determine whether a mixture, based mainly on ground bitter almonds mixed in a cup of water (the "Mixture"), had any glucose lowering effect, at meal-time, in subjects with Type-2 diabetes, maintained on Metformin and diet, or diet alone. It was assumed that these natural ingredients can be used safely and will improve post-prandial blood glucose levels.

### Study Design

Type 2 diabetic subjects (7 males and 3 females) with HgA1c between 6.5-8.5%, BMI < 32, generally healthy individuals, volunteered for this study. After the initial screening visit, subjects attended the Diabetes Unit twice. In each visit they received the same standard breakfast. Blood samples for glucose evaluation, were withdrawn at timed intervals (every 10 minutes for the first two hr. and every 30 min for the following 2 hr.), up to 4 hours following the ingestion of the meal with the Mixture, given at the end of the meal. On 1^{st} visit the volunteers received the active Mixture, the "placebo" of other natural ingredients (based on sweet almonds), was administered on the 2^{nd} visit.

### Results

A significant decrease in post-prandial blood glucose levels, was demonstrated following the ingestion of the Mixture compared to the placebo. In six subjects the response rate was much more pronounced while in four individuals the response was less impressive.

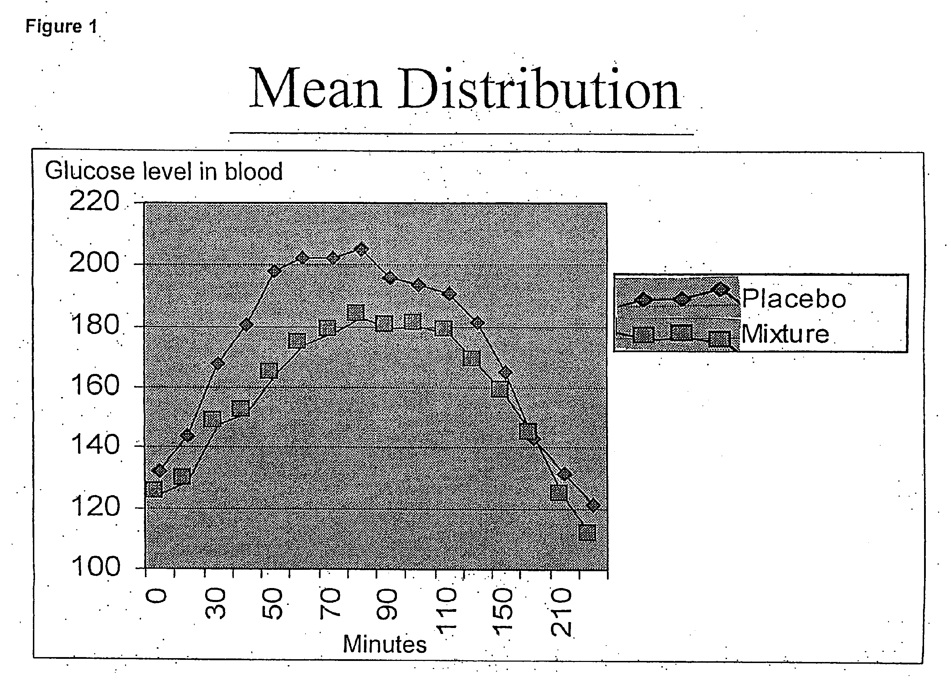

Fig 1 is a graphical representation of the mean distribution of glucose levels in blood as a function of time after the administration of a composition according to the present invention and administration of a placebo and depicts the results of 6 responders.

### Conclusions

In Type 2 diabetic patients, the administration of a mixture (based on natural ingredients containing ground bitter almonds) was effective in attenuating post-prandial blood glucose levels.

### Example 5: Metabolic profile of bitter and sweet almonds

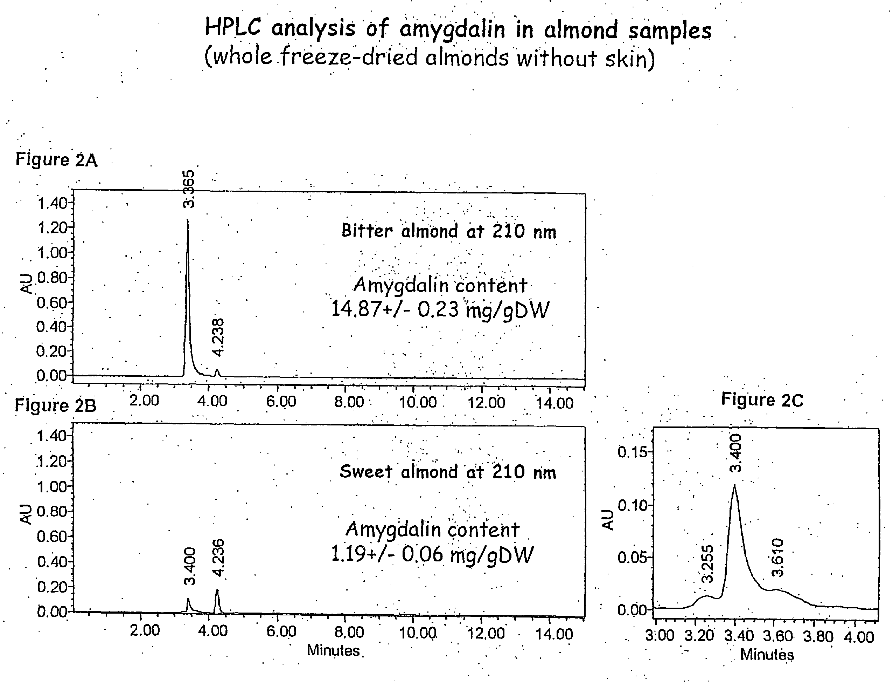

In order to determine the active components in bitter almonds the volatile and non-volatile compounds were compared by GC-MS (Gas chromatography - Mass spectrum) and HPLC (High performance liquid chromatography). Figures 2A and 2B respectively show the HPLC chromatograms comparing bitter and sweet almonds, and Figure 2C shows the HLPC chromatogram of a standard commercial amydalin.

### Determination of amygdalin content in bitter and sweet almonds

0.5g of crushed almonds were overnight extracted with 10ml methanol. Isocratic mobile phase was 85% ACN and 15% water, flow of 0.7m1/min. Column C18 NovaPak 4mm, 4.6x250mm.

Amygdalin content was calculated using standard curve of authentic standard as shown in Figure 2C.

### Results

A nearly 9 folds higher amygdalin peak was detected in the bitter compared to sweet almond seeds. (Fig 2A, Fig 2B).

### Example 6: Extraction of Volatiles in bitter and sweet almonds.

### Water treatment and MTBE

It is well established that almonds contain hydrolytic enzymes that cleave amygdalin into other products. To allow for the enzymes to properly work, the ground powders were incubated with water as follows: 0.2 gram of each mix (1-5) was incubated at room temperature with 1 ml of H₂O (2 replicates) vigorously shaken at 200 RPM. Then, 10 ml of MTBE containing 10 µg of *iso*-butyl benzene as an internal standard was added to each sample. The samples were shaken vigorously (200 RPM) for 5 h at room temperature. The upper MTBE layer was separated and dried by passing through a small column (Pasteur pipette) containing sodium sulfate, and then concentrated to a volume of 1 ml under a gentle stream of nitrogen. The results are presented as µg compound /gr fw. in the following table 1.

**Table 1 - Water Treatment with MTBE**

| avg µg compound/gr fw. | 1+w | 2+ws | 3+ws | 4+w | 5+w | identification |
|---|---|---|---|---|---|---|
| Aldehyde | | | | | | |
| 2,4, decadienal | 50,85 | 4,05 | 27,17 | 3,46 | 1,87 | MS, KI |
| 2-Decenal, <E-> | 143,62 | 6,11 | 107,81 | 6,80 | 1,54 | MS, KI |
| Benzaldehyde | 60,77 | 6.091,01 | 60,84 | 4.309,60 | 170,49 | MS, KI |
| Cinnamaldehyde <Z> | 64,83 | - | 104,11 | - | - | MS, KI |
| Cinnamaldehyde <E-> | 3.005,26 | 3,98 | 3.160,69 | 8,44 | 4,23 | MS, KI |
| decadienal 2e, 4e | 41,83 | 2,38 | 25,96 | 2,27 | 0,79 | MS, KI |
| E-2-Z-6-Nonadienal | 0,52 | - | - | - | 8,80 | MS, KI |
| Methoxy cinnamaldehye<(E)- | 107,99 | - | 85,26 | - | - | KI-D |
| Methoxy cinnamic aldehyde <(Z)> | 25,03 | - | 44,43 | 0,29 | - | MS, KI |

| Aromatic Alcohols | | | | | | |
|---|---|---|---|---|---|---|
| Benzyl alcohol | - | 219,19 | - | 78,35 | 24,73 | MS, KI |
| cinamyl alcohol | 19,22 | - | 32,00 | 1,61 | - | MS, KI |
| Phenethyl alcohol | 2,57 | 2,56 | 2,35 | 1,01 | 7,78 | MS, KI |

| Phenol | | | | | | |
|---|---|---|---|---|---|---|
| 2-Methoxy-4-vinylphenol | 3,50 | 3,07 | - | 1,26 | 16,33 | MS |
| Coumarin | 48,99 | - | 45,19 | 0,41 | 0,49 | MS, KI |
| Eugenol | - | - | - | 4,25 | 7,44 | MS, KI |
| 4-vinyl phenol | 6,71 | 3,71 | 1,07 | 3,55 | 31,96 | MS |

| Monoterpenes | | | | | | |
|---|---|---|---|---|---|---|
| alpha- Pinene | - | 61,99 | 6,07 | - | - | MS, KI |
| BETA.- Pinene | 16,64 | 176,23 | 32,75 | - | - | MS, KI |
| para -Cymene | 6,91 | 47,93 | 9,27 | - | - | MS, KI |
| Limonene | 35,01 | 325,71 | 51,57 | 1,57 | - | MS, KI |
| Limonene oxide e | 7,26 | 10,58 | 4,55 | - | - | MS, KI |
| gamma - Terpinene | - | 44,56 | 5,70 | 0,66 | - | MS, KI |
| Carveol e | - | 10,94 | - | - | - | MS, KI |
| alpha -Terpineol | 6,62 | 12,02 | 7,21 | 3,96 | 1,56 | MS, KI |

| Sesquiterpenes | | | | | | |
|---|---|---|---|---|---|---|
| beta-Elemene | - | 7,03 | - | - | - | MS, KI |
| alpha- Bergamotene | 3,00 | 18,05 | 3,59 | 3,48 | - | MS, KI |
| Beta-Bisabolene | 6,74 | 25,20 | 6,94 | 7,15 | 1,76 | MS, KI |
| Caryophyllene-<E-> | 3,35 | 12,40 | 3,87 | 3,48 | - | MS, KI |
| Alpha-Copaene | 28,87 | - | 26,57 | - | - | MS, KI |
| Beta-Cubebene | 8,27 | - | 7,90 | - | - | MS, KI |
| Curcumene<AR-> | 8,76 | - | 5,15 | - | - | MS, KI |
| gamma -Elemene | - | 3,52 | - | - | - | MS, KI |
| alpha- Famesene | - | - | 16,26 | 7,34 | - | MS, KI |
| MUUROLA-3,5-DIENE<(Z)> | 4,56 | - | 3,71 | - | - | MS, KI |
| MUUROLA-3,5-DIENE<E-> | - | - | 8,27 | - | - | |
| MUUROLA-4(14),5-DIENE<E-> | 5,08 | - | 4,59 | - | - | MS, KI |
| alpha-Muurolene | 29,41 | - | 25,75 | - | - | MS, KI |
| gamma -Muurolene | 9,14 | - | 8,36 | - | - | MS, KI |
| unknown sesquiterpens | 7,05 | - | 6,51 | - | - | |
| Calamenene<(Z)> | 15,87 | - | 14,00 | - | - | MS, KI |
| Cubenol | 7,33 | 2,72 | 8,19 | - | - | MS, KI |
| t-Muurolol | 8,15 | - | 9,05 | - | - | MS, KI |
| | | | | | | |
| Mandelamide | | - 181,08 | - | 41,40 | 227,78 | MS |

| | | | | | | |
|---|---|---|---|---|---|---|
| *MS- Mass spectrum, KI-Kovach index, KI-D- no match to Kovach index | | | | | | |

### Example 7: Extraction with MTBE only

As controls (without water incubation, 1 ml of (MTBE) was added instead of the water. The same procedure was followed as above. For this experiment, only one replication per sample was performed.

| Mix no. | Extraction-1 | Extraction-2 | |
|---|---|---|---|
| 1 | W+MTBE | MTBE | Sweet |
| 2 | W+MTBE | MTBE | Bitter |
| 3 | W+MTBE | MTBE | Sweet |
| 4 | W+MTBE | MTBE | Bitter |
| 5 | W+MTBE | MTBE | Leaf extract |

A 1-µl aliquot of the concentrated MTBE was injected into an Agilent GC-MSD system (CA, USA). The instrument was equipped with Rtx-5 SIL column (30 m length x 0.25 mm i.d., 0.25 µm film thickness, stationary phase 95% dimethyl- 5% diphenyl polysiloxane). Helium (0.8 ml/min) was used as a carrier gas with splitless injection. The injector temperature was 250 °C, and the detector temperature was 280°C. The conditions used were as follows: initial temperature was 50 °C for 1 min followed by a ramp of 50 to 260 °C at a rate of 5 °C/min, and additional 5 min at 260 °C. A quadrupole mass detector with electron ionization at 70 eV was used to acquire the MS data in the range of 41 to 350 m/z. A mixture of straight-chain alkanes (C7-C23) was injected into the column under the above-mentioned conditions for retention indices calculation. The identification of the volatiles was assigned by comparison of their retention indices with those of standards injected under the same conditions.

The results are presented in table 2 hereinafter.

**Table 2 Extraction with MTBE only**

| µg compound/gr fw. | 1 | 2 | 3 | 4 | 5 |
|---|---|---|---|---|---|
| Aldehyde | | | | | |
| 2,4, decadienal | 101,73 | 0,68 | 44,20 | 5,96 | 1,15 |
| 2-Decenal, <E-> | 230,26 | 1,21 | 136,12 | 21,78 | 3,22 |
| Benzaldehyde | 17,88 | 4,53 | 11,43 | 17,83 | - |
| Cinnamaldehyde <Z> | 15,75 | - | 2,89 | - | - |
| Cinnamaldehyde <E-> | 1.039,55 | 1,17 | 938.57 | 6,37 | - |
| decadienal 2e, 4e | 77,39 | 0,38 | 38,57 | 4,87 | 0,69 |
| E-2-Z-6-Nonadienal | - | - | - | - | - |
| Methoxy cinnamaldehye<(E)- | 21,33 | - | 23,15 | - | - |
| Methoxy cinnamic aldehyde <(Z)> | 6,98 | - | - | - | - |

| Aromatic Alcohols | | | | | |
|---|---|---|---|---|---|
| Benzyl alcohol | - | - | - | 7,60 | 1,89 |
| cinamyl alcohol | 5,62 | - | 6,19 | 1,69 | - |
| Phenethyl alcohol | - | - | - | - | 1,45 |

| Phenol | | | | | |
|---|---|---|---|---|---|
| 2-Methoxy-4-vinylphenol | - | 0,25 | - | 0,59 | 3,77 |
| Coumarin | 8,24 | - | 7,40 | - | - |
| Eugenol | - | - | - | 6,01 | 5,02 |
| 4-vinyl phenol | - | - | - | 1,58 | - |

| Monoterpenes | | | | | |
|---|---|---|---|---|---|
| alpha- Pinene | - | 36,87 | - | - | - |
| BETA.- Pinene | 24,01 | 115,93 | 18,64 | - | - |
| para -Cymene | 12,80 | 32,16 | 7,43 | - | - |
| Limonene | 39,78 | 219,12 | 34,48 | 5,39 | - |
| Limonene oxide e | 10,72 | 7,25 | 5,98 | - | - |
| gamma - Terpinene | - | 28,26 | - | 0,95 | - |
| Carveol e | - | 1,75 | - | - | - |
| alpha -Terpineol | - | 6,11 | 3,15 | 4,45 | 2,09 |

| Sesquiterpenes | | | | | |
|---|---|---|---|---|---|
| beta-Elemene | 5,41 | 3,90 | 4,26 | - | - |
| alpha- Bergamotene | 3,31 | 11,39 | 2,39 | 5,36 | 2,08 |
| Beta-Bisabolene | 6,85 | 16,35 | 4,91 | 12,22 | 6,94 |
| Caryophyllene-<E-> | 4,68 | 9,70 | 4,18 | 5.63 | 2,14 |
| Alpha-Copaene | 29,61 | - | 19,36 | 0,32 | - |
| Beta-Cubebene | - | - | 4,59 | - | - |
| Curcumene<AR-> | 5,33 | - | - | - | - |
| gamma -Elemene | - | 2,03 | - | 0,60 | - |
| alpha- Famesene | - | 10,66 | - | - | - |
| MUUROLA-3,5-DIENE<(Z)> | 4,41 | - | 2,57 | - | - |
| MUUROLA-3,5-DIENE<E-> | - | - | 3,17 | - | - |
| MUUROLA-4(14),5-DIENE<E-> | 5,44 | - | 2,59 | - | - |
| alpha-Muurolene | 30,18 | - | 19,33 | - | - |
| gamma -Muurolene | 9,25 | - | - | - | - |
| unknown sesquiterpens | 7,00 | - | 5,35 | - | - |
| Calamenene<(Z)> | 17,06 | - | 9,99 | - | - |
| Cubenol | 6,94 | 1,48 | 4,64 | 2,37 | - |
| t-Muurolol | 7,55 | - | 7,10 | - | - |
| | | | | | |
| Mandelamide | - | - | - | 4,23 | - |

### Results

- A much higher level of volatiles were obtained when the mixes were pre incubated in H₂O for 1 hr as compared to those non-treated with water prior to extraction. The compositions between the samples also varied (see tables).
- Volatile aldehydes are known to be the major group contributing to almond mixture flavor. Among them cinnamaldehyde (cinnamon aroma), benzaldehyde (almonds aroma), methoxy cinnamaldehyde<(E)-(anisaldehyde, coumarin aroma ) were identified in bitter samples in high levels and in much lower levels in sweet almonds. The typical almond flavor compound benzaldehyde was detected at very high levels in (up to 6000 gg/gfw) bitter almonds and in lower amounts (∼60 µg/gfw) but being still major components in sweet almonds.
- Other aromatic alcohols such as benzyl and cinnamyl alcohol and mandelamide (a degradation product of amygdalin) were present only in bitter almonds and leaves, and absent in sweet almonds.
- The most prominent constituents in sweet almonds were aldehydes mostly cinnamaldehyde and his derivates, as well as cinnamyl alcohol and a variety of sesquiterpenes.
- The second most prominent group (especially in the sweet almonds but also prominent in bitter almonds) are the terpenes- that included monoterpenes (Cymene-para" Limonene, Limonene oxide e, Terpinene-gamma, Carveol e, Terpineol-alpha) and sesquiterpenes.
- The major volatiles identified in Mix 5 (leaf extract) were benzaldehyde, benzyl alcohol, 2-Methoxy-4-vinylphenol, 4-vinyl phenol and mandelamide.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description.

## Claims

1. Use of a natural source of at least one cyanogenic glucoside, wherein the natural source is obtainable from kernels of bitter almonds as active ingredient for the manufacture of a pharmaceutical composition for alleviating excess levels of sugar in the blood of type 2 diabetic patients, wherein the pharmaceutical composition comprises a powder formed from a mixture of dry bitter almond kernels and from a dried lemon component selected from the group consisting of lemon pulp; peel; and seeds and combinations thereof.

2. Use according to claim 1 wherein a further natural source of at least one cyanogenic glucoside is from kernels from fruits of the Prunus species.

3. Use according to claim 1 wherein a further natural source of at least one cyanogenic glucoside is from the powder formed from a dried lemon component selected from the group consisting of lemon pulp; peel; and seeds and combinations thereof.

4. Use according to claim 1 wherein said powder comprises between about 70% and 90% dried and ground bitter almond kernels; and between about 30% and 10% of a dried lemon component selected from the group consisting of lemon pulp; peel; and seeds and combinations thereof.

5. Use according to any one of the preceding claims wherein the pharmaceutical composition further comprises at least one component from 2,4, decadienal; (E)-2-decenal; benzaldehyde; (Z)-cinnamaldehyde; (E)-cinnamaldehyde; (2E, 4E)-decadienal; (2E, 6Z)-nonadienal; (E)-methoxy cinnamaldehyde; (Z)-methoxy cinnamic aldehyde; benzyl alcohol; cinamyl alcohol; phenethyl alcohol; 2-methoxy-4-vinylphenol; coumarin; eugenol; 4-vinyl phenol; alpha-pinene; beta-pinene; para-cymene; limonene; (E)-limonene oxide; gamma-terpinene; (E)-carveol; alpha-terpineol; beta-elemene; alpha-bergamotene; beta-bisabolene; (E)-caryophyllene; alpha-copaene; beta-cubebene; (A,R)-curcumene; gamma-elemene; alpha-farnesene; (Z)-muurola-3,5-diene; (E)-muurola-3,5-diene; (E)-muurola-4(14),5-diene; alpha-muurolene; gamma-muurolene; other sequiterpens; (Z)-calamenene; cubenol; t-muurolol; mandelamide.

6. A mixture, based mainly on ground bitter almonds mixed in water for use in alleviating excess levels of sugar in the blood of type 2 diabetic patients.

7. Use of ground bitter almonds for the manufacture of a pharmaceutical composition for alleviating excess levels of sugar in the blood of type 2 diabetic patients, wherein the pharmaceutical composition is a mixture, based mainly on ground bitter almonds mixed in water.

## Patentansprüche

1. Verwendung einer natürlichen Quelle mindestens eines cyanogenen Glukosids, wobei die natürliche Quelle aus Kernen von Bittermandeln als Wirkstoff für die Herstellung einer pharmazeutischen Zusammensetzung für die Senkung übermäßiger Zuckerspiegel im Blut von Patienten mit Typ-2-Diabetes erhalten werden kann, wobei die pharmazeutische Zusammensetzung ein Pulver aufweist, das aus einem Gemisch aus trockenen Bittermandelkernen und aus einer getrockneten Zitronenkomponente gebildet ist, welche aus der Gruppe ausgewählt ist, die aus Zitronenfruchtfleisch, -schale und -samen und Kombinationen davon besteht.

2. Verwendung nach Anspruch 1, wobei eine weitere natürliche Quelle mindestens eines cyanogenen Glukosids aus Kernen von Früchten der Gattung Prunus stammt.

3. Verwendung nach Anspruch 1, wobei eine weitere natürliche Quelle mindestens eines cyanogenen Glukosids aus dem Pulver stammt, das aus einer getrockneten Zitronenkomponente gebildet ist, welche aus der Gruppe ausgewählt ist, die aus Zitronenfruchtfleisch, -schale und -samen und Kombinationen davon besteht.

4. Verwendung nach Anspruch 1, wobei das Pulver zwischen etwa 70 % und 90 % getrocknete und gemahlene Bittermandelkerne und zwischen etwa 30 % und 10 % einer getrockneten Zitronenkomponente aufweist, die aus der Gruppe ausgewählt ist, die aus Zitronenfruchtfleisch, -schale und -samen und Kombinationen davon besteht.

5. Verwendung nach einem der vorherigen Ansprüche, wobei die pharmazeutische Zusammensetzung des Weiteren mindestens eine Komponente aus 2,4-Decadienal, (E)-2-Decenal; Benzaldehyd; (Z)-Zimtaldehyd; (E)-Zimtaldehyd; (2E, 4E)-Decadienal; (2E,6Z)-Nonadienal; (E)-Methoxyzimtaldehyd; (Z)-Methoxyzimtaldehyd; Benzylalkohol; Zimtalkohol; Phenethylalkohol; 2-Methoxy-4-vinylphenol; Coumarin; Eugenol; 4-Vinylphenol; Alpha-Pinen; Beta-Pinen; Para-Cymen; Limonen; (E)-Limonenoxide; Gamma-Terpinen; (E)-Carveol; Alpha-Terpineol; Beta-Elemen; Alpha-Bergamoten; Beta-Bisabolen; (E)-Caryophyllen; Alpha-Copaen; Beta-Cubeben; (A,R)-Curcumen; Gamma-Elemen; Alpha-Farnesen; (Z)-Muurola-3,5-dien; (E)-Muurola-3,5-dien; (E)-Muurola-4(14),5-dien; Alpha-Muurolen; Gamma-Muurolen; anderen Sequiterpenen; (Z)-Calamenen; Cubenol; t-Muurolol; Mandelamid aufweist.

6. Gemisch, das hauptsächlich auf gemahlenen Bittermandeln basiert, die in Wasser gemischt sind, für die Anwendung zum Senken übermäßiger Zuckerspiegel im Blut von Patienten mit Typ-2-Diabetes.

7. Verwendung gemahlener Bittermandeln für die Herstellung einer pharmazeutischen Zusammensetzung zum Senken übermäßiger Zuckerspiegel im Blut von Patienten mit Typ-2-Diabetes, wobei die pharmazeutische Zusammensetzung ein Gemisch ist, welches hauptsächlich auf gemahlenen Bittermandeln, gemischt in Wasser, basiert.

## Revendications

1. Utilisation d'une source naturelle d'au moins un glucoside cyanogène, la source naturelle pouvant être obtenue à partir de noyaux d'amandes amères comme ingrédient actif pour la fabrication d'une composition pharmaceutique pour pallier les niveaux excessifs de sucre dans le sang de patients diabétiques de type 2, la composition pharmaceutique comprenant une poudre formée à partir d'un mélange de noyaux d'amandes amères séchés et à partir d'un composant de citron séché choisi dans le groupe constitué par la pulpe, la peau et les graines de citron et leurs combinaisons.

2. Utilisation selon la revendication 1, dans laquelle une autre source naturelle d'au moins un glucoside cyanogène est obtenue à partir de noyaux de fruits de l'espèce Prunus.

3. Utilisation selon la revendication 1, dans laquelle une autre source naturelle d'au moins un glucoside cyanogène est obtenue à partir de la poudre formée à partir d'un composant de citron séché choisi dans le groupe constitué par la pulpe, la peau et les graines de citron, et leurs combinaisons.

4. Utilisation selon la revendication 1, dans laquelle ladite poudre comprend entre environ 70 % et 90 % de noyaux d'amandes amères séchés et moulus, et entre environ 30 % et 10 % d'un composant de citron séché choisi dans le groupe constitué par la pulpe, la peau et les graines de citron, et leurs combinaisons.

5. Utilisation selon l'une quelconque des revendications précédentes, dans laquelle la composition pharmaceutique comprend en outre au moins un composant choisi parmi le 2,4-décadénial ; le (E)-2-décénal ; le benzaldéhyde ; le (Z)-cinnamaldéhyde ; le (E)-cinnamaldéhyde ; le (2E,4E)-décadiénal ; le (2E-6Z)-nonadiénal ; le (E)-méthoxy cinnamaldéhyde ; le (Z)-aldéhyde méthoxycinnamique ; l'alcool benzylique ; l'alcool cinnamylique ; l'alcool phénéthylique ; le 2-méthoxy-4-vinylphénol ; la coumarine ; l'eugénol ; le 4-vinylphénol ; l'alpha-pinène ; le bêta-pinène ; le paracymène ; le limonène ; l'oxyde de (E)-limonène ; le gamma-terpinène ; le (E)-carvéol ; l'alpha-terpinéol ; le bêta-élémène ; l'alpha-bergamotène ; le bêta-bisabolène ; le (E)-caryophyllène ; l'alpha-copaène ; le bêta-cubébène ; le (A,R)-curcumène ; le gamma-élémène ; l'alpha-farnésène ; le (Z)-muurola-3,5-diène ; le (E)-muurola-3,5-diène ; le (E)-muurola-4(14),5-diène ; l'alpha-muurolène ; le gamma-muurolène ; d'autres sesquiterpènes ; le (Z)-calaménène ; le cubénol ; le t-muurolol ; le mandélamide.

6. Mélange, fondé principalement sur des amandes amères moulues mélangées avec de l'eau pour une utilisation pour pallier les niveaux excessifs de sucre dans le sang de patients diabétiques de type 2.

7. Utilisation d'amandes amères moulues pour la fabrication d'une composition pharmaceutique pour pallier les niveaux excessifs de sucre dans le sang de patients diabétiques de type 2, dans laquelle la composition pharmaceutique est un mélange, fondé principalement sur des amandes amères moulues mélangées avec de l'eau.
